# EUROPEAN PATENT APPLICATION

(11) **EP 4 653 428 A1**
(43) Date of publication of application: **26.11.2025**
(21) Application number: 24744389.8
(22) Date of filing: 19.01.2024
(51) Int. Cl.: C07D 401/04, C07D 401/14, A61K 31/4439, A61P 1/16, A61P 3/00, A61P 9/00, A61P 11/00, A61P 13/00, A61P 25/00, A61P 27/00, A61P 35/00

(54) **PHARMACEUTICALLY ACCEPTABLE SALT AND CRYSTAL FORM OF TRIAZOLE COMPOUND, AND PREPARATION METHOD THEREFOR**

(30) Priority: 19.01.2023 CN 202310066282
(71) Applicant: Jiangsu Hengrui Pharmaceuticals Co., Ltd., Lianyungang, Jiangsu 222047 (CN)
(72) Inventor: JIA, Lina, Lianyungang, Jiangsu 222047 (CN); WANG, Jie, Lianyungang, Jiangsu 222047 (CN); YANG, Junran, Lianyungang, Jiangsu 222047 (CN); DU, Zhenxing, Lianyungang, Jiangsu 222047 (CN); WANG, Jie, Lianyungang, Jiangsu 222047 (CN)
(74) Representative: dompatent
(86) International application number: PCT/CN2024/073211
(87) International publication number: WO 2024/153219

(57) **Abstract**

The present disclosure relates to a pharmaceutically acceptable salt and a crystal form of a triazole compound, and a preparation method therefor. Specifically, provided in the present disclosure are a pharmaceutically acceptable salt and a crystal form of the compound (1S,3aS,5S,6aR)-5-((6-(5-(((4-(methoxymethyl)pyrimidin-2-yl)oxy)methyl)-1-methyl-1*H*-1,2,3-triazol-4-yl)-2-methylpyridin-3-yl)oxy) octahydrocyclopentadien-1-carboxylic acid of formula I, and a preparation method therefor. The corresponding salt has a good stability and can be better used in clinical treatment.

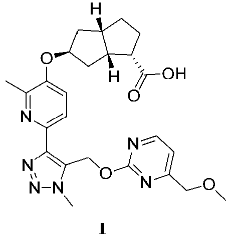

## Description

The present application claims priority to Chinese Patent Application No. 2023100662824 filed on January 19, 2023, which is incorporated herein by reference in its entirety.

### TECHNICAL FIELD

The present disclosure pertains to the field of pharmaceutics and relates to a pharmaceutically acceptable salt and a crystal form of a triazole compound and a preparation method therefor.

### BACKGROUND

Blood phospholipids are membrane-derived bioactive lipid mediators, and the most important blood phospholipid in medicine is lysophosphatidic acid (LPA). Lysophospholipids affect fundamental cellular functions including proliferation, differentiation, survival, migration, adhesion, invasion, and morphogenesis. These functions influence many biological processes that include, but are not limited to, neurogenesis, angiogenesis, wound healing, fibrosis, immunity, and carcinogenesis.

LPA is not a single molecular entity but a group of endogenous structural variants of fatty acids that vary in length and saturation. The structural backbone of LPA is derived from a glycerol-based phospholipid, such as phosphatidylcholine (PC) or phosphatidic acid (PA). Lysophosphatidic acid (LPA) is a lysophospholipid that acts through sets of specific G protein-coupled receptors (GPCRs) in an autocrine and paracrine fashion. LPA binds to its cognate GPCRs (LPA1, LPA2, LPA3, LPA4, LPA5, and LPA6), activating intracellular signaling pathways to produce a variety of biological responses. Antagonists of the LPA receptors find use in the treatment of diseases, disorders, or conditions in which LPA plays a role.

PCT/CN2022/106706 discloses a class of novel triazole derivatives, among which the derivative represented by formula I is chemically named (1S,3aS,5S,6aR)-5-((6-(5-(((4-(methoxymethyl)pyrimidin-2-yl)oxy)methyl)-1-methyl-1*H*-1,2,3-triazol-4-yl)-2-methylpyridin-3-yl)oxy)octahydropentalene-1-carboxylic acid, and its use as an LPA1 inhibitor is demonstrated. PCT/CN2022/106706 is incorporated herein by reference in its entirety.

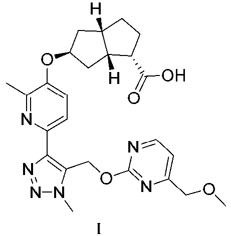

The structure of a crystal form used as a pharmaceutically active ingredient often affects the chemical and physical stabilities of the drug. Changes in crystallization and storage conditions may cause changes in the crystal structure of the compound and sometimes the generation of other crystal forms. Generally, amorphous drug products have no regular crystal structure and often have other defects, such as poor product stability, difficult filtration, proneness to agglomeration, and poor flowability. Therefore, the research on pharmaceutically acceptable salts and crystal forms of the compound represented by formula I, chemically named (1S,3aS,5S,6aR)-5-((6-(5-(((4-(methoxymethyl)pyrimidin-2-yl)oxy)methyl)-1-methyl-1*H*-1,2,3-triazol-4-yl)-2-methylpyridin-3-yl)oxy)octahydropentalene-1-carboxylic acid, is of great significance for the development of drugs that are suitable for industrial production and have good bioactivity.

### SUMMARY

In one aspect, the present disclosure provides a crystal form A of the compound of formula I, (1S,3aS,5S,6aR)-5-((6-(5-(((4-(methoxymethyl)pyrimidin-2-yl)oxy)methyl)-1-methyl-1*H*-1,2,3-triazol-4-yl)-2-methylpyridin-3-yl)oxy)octahydropentalene-1-carboxylic acid.

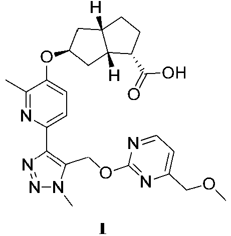

In some embodiments, an X-ray powder diffraction pattern expressed in terms of 2*θ* angles, which are diffraction angles, of the crystal form A has characteristic peaks at 7.534, 10.223, 14.364, 17.269, 18.495, and 19.553.

In some other embodiments, the X-ray powder diffraction pattern expressed in terms of 2*θ* angles, which are diffraction angles, of the crystal form A has characteristic peaks at 7.534, 10.223, 14.364, 15.816, 16.678, 17.269, 18.495, and 19.553.

In some other embodiments, the X-ray powder diffraction pattern expressed in terms of 2*θ* angles, which are diffraction angles, of the crystal form A has characteristic peaks at 7.534, 10.223, 14.364, 15.816, 16.678, 17.269, 18.495, 19.553, 21.043, 21.342, 23.647, and 25.965.

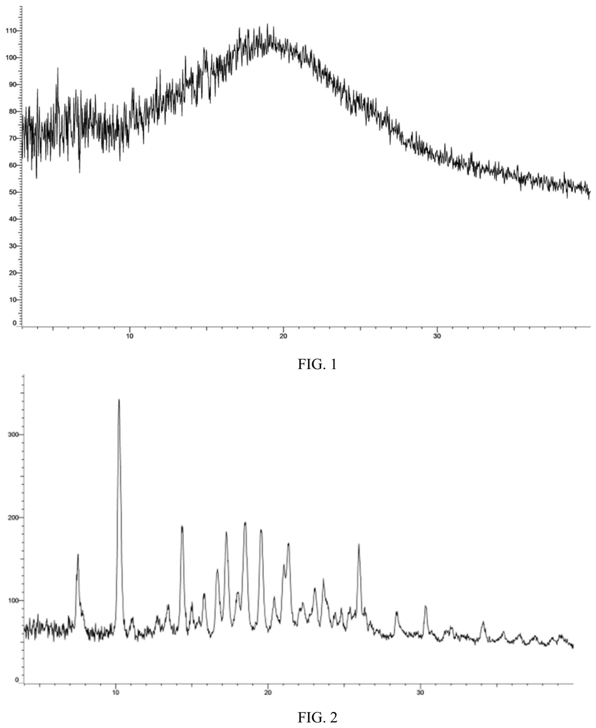

Most preferably, the X-ray powder diffraction pattern expressed in terms of 2*θ* angles, which are diffraction angles, of the crystal form is shown in FIG. 2.

In another aspect, the present disclosure provides a preparation method for the crystal form A of the compound of formula I, comprising: dissolving the compound of formula I in propylene glycol methyl ether, adding n-heptane, and stirring for crystallization.

In some other embodiments, the preparation method for the crystal form A of the compound of formula I comprises: dissolving the compound of formula I in a solvent (1), adding a solvent (2), adding a solvent (3), and stirring for crystallization, wherein the solvent (1) is at least one solvent selected from the group consisting of propylene glycol methyl ether, 1,4-dioxane, 10% water/acetone, N,N-dimethylformamide, N,N-dimethylacetamide, 50% methanol/chloroform, and 67% tetrahydrofuran/ethanol; the solvent (2) is selected from the group consisting of methyl tert-butyl ether; the solvent (3) is selected from the group consisting of n-heptane.

In some other embodiments, the preparation method for the crystal form A of the compound of formula I comprises: dissolving the compound of formula I in acetone and stirring for crystallization.

In another aspect, the present disclosure provides a crystal form B of the compound of formula I, (1S,3aS,5S,6aR)-5-((6-(5-(((4-(methoxymethyl)pyrimidin-2-yl)oxy)methyl)-1-methyl-1*H*-1,2,3-triazol-4-yl)-2-methylpyridin-3-yl)oxy)octahydropentalene-1-carboxylic acid.

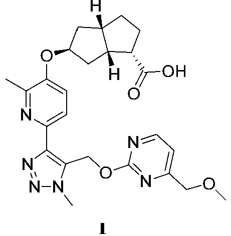

In some embodiments, an X-ray powder diffraction pattern expressed in terms of 2*θ* angles, which are diffraction angles, of the crystal form B has characteristic peaks at 7.437, 10.381, 14.955, and 17.513.

In some other embodiments, the X-ray powder diffraction pattern expressed in terms of 2*θ* angles, which are diffraction angles, of the crystal form B has characteristic peaks at 7.437, 10.381, 14.717, 14.955, 17.513, 21.395, 22.607, and 23.221.

In some other embodiments, the X-ray powder diffraction pattern expressed in terms of 2*θ* angles, which are diffraction angles, of the crystal form B has characteristic peaks at 7.437, 10.381, 14.717, 14.955, 17.513, 18.466, 21.395, 21.692, 22.607, 23.221, and 25.972.

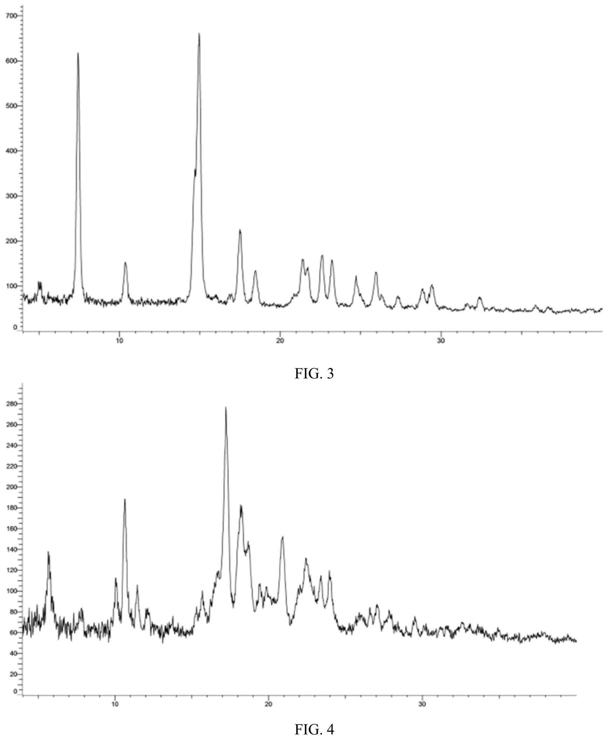

In some other embodiments, the X-ray powder diffraction pattern expressed in terms of 2*θ* angles, which are diffraction angles, of the crystal form B is shown in FIG. 3.

In another aspect, the present disclosure provides a preparation method for the crystal form B of the compound of formula I, comprising: dissolving the compound of formula I in a solvent (A), adding a solvent (B), adding a solvent (C), and stirring for crystallization, wherein the solvent (A) is at least one solvent selected from the group consisting of tetrahydrofuran, dichloromethane, 10% water/isopropanol, and trichloromethane; the solvent (B) is selected from the group consisting of methyl tert-butyl ether; the solvent (C) is selected from the group consisting of n-heptane.

In some other embodiments, the preparation method for the crystal form B of the compound of formula I comprises: dissolving the compound of formula I in water, methanol, ethanol, isopropanol, n-propanol, ethyl acetate, acetonitrile, isopropyl acetate, methyl tert-butyl ether, 2-butanone, methyl isobutyl ketone, n-heptane, isopropyl ether, isoamyl alcohol, 10% water/methanol, 7% water/ethanol, 50% methanol/water, 50% ethyl acetate/ethanol, 50% ethyl acetate/n-heptane, cyclohexane, n-hexane, or toluene, and stirring for crystallization.

In another aspect, the present disclosure provides a crystal form C of the compound of formula I, (1S,3aS,5S,6aR)-5-((6-(5-(((4-(methoxymethyl)pyrimidin-2-yl)oxy)methyl)-1-methyl-1*H*-1,2,3-triazol-4-yl)-2-methylpyridin-3-yl)oxy)octahydropentalene-1-carboxylic acid.

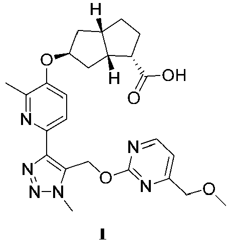

In some embodiments, an X-ray powder diffraction pattern expressed in terms of *2θ* angles, which are diffraction angles, of the crystal form C has characteristic peaks at 5.702, 10.643, 17.229, 18.213, 18.666, and 20.900.

In some other embodiments, the X-ray powder diffraction pattern expressed in terms of 2*θ* angles, which are diffraction angles, of the crystal form C has characteristic peaks at 5.702, 10.074, 10.643, 11.463, 17.229, 18.213, 18.666, 20.900, 22.422, and 23.968.

In some other embodiments, the X-ray powder diffraction pattern expressed in terms of 2*θ* angles, which are diffraction angles, of the crystal form C has characteristic peaks at 5.702, 10.074, 10.643, 11.463, 16.738, 17.229, 18.213, 18.666, 20.900, 22.057, 22.422, 23.397, and 23.968.

In some other embodiments, the X-ray powder diffraction pattern expressed in terms of 2*θ* angles, which are diffraction angles, of the crystal form C is shown in FIG. 4.

In another aspect, the present disclosure further provides a pharmaceutically acceptable salt of the compound represented by formula I, (1S,3aS,5S,6aR)-5-((6-(5-(((4-(methoxymethyl)pyrimidin-2-yl)oxy)methyl)-1-methyl-1*H*-1,2,3-triazol-4-yl)-2-methylpyridin-3-yl)oxy)octahydropentalene-1-carboxylic acid, wherein the pharmaceutically acceptable salt is selected from the group consisting of a sodium salt and an ethanolamine salt.

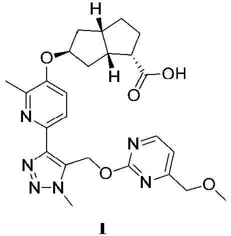

In an optional embodiment, the chemical ratio of the compound of formula I, (1S,3aS,5S,6aR)-5-((6-(5-(((4-(methoxymethyl)pyrimidin-2-yl)oxy)methyl)-1-methyl-1*H*-1,2,3-triazol-4-yl)-2-methylpyridin-3-yl)oxy)octahydropentalene-1-carboxylic acid to sodium ions is 1:1.

The present disclosure further provides a preparation method for a pharmaceutically acceptable salt of the compound of formula I, (1S,3aS,5S,6aR)-5-((6-(5-(((4-(methoxymethyl)pyrimidin-2-yl)oxy)methyl)-1-methyl-1*H*-1,2,3-triazol-4-yl)-2-methylpyridin-3-yl)oxy)octahydropentalene-1-carboxylic acid, comprising a step of reacting the compound of formula I with a base, wherein the base is selected from the group consisting of sodium hydroxide and ethanolamine.

The solvent used for salt formation in the present disclosure is one or more solvents selected from the group consisting of, but not limited to, acetonitrile, ethanol/ethyl acetate, methanol/acetonitrile, 2-butanone, isopropanol, isopropyl acetate, n-propanol, n-heptane, acetone, tetrahydrofuran, ethanol, methanol, 1,4-dioxane, dichloromethane/methanol, water/isopropanol, and tetrahydrofuran/ethanol.

Further, in an optional embodiment, the method for preparing the aforementioned pharmaceutically acceptable salt further comprises such a step as crystallization, filtration, washing, or drying.

In another aspect, the present disclosure further provides a crystal form I of a sodium salt of the compound of formula I, (1S,3aS,5S,6aR)-5-((6-(5-(((4-(methoxymethyl)pyrimidin-2-yl)oxy)methyl)-1-methyl-1*H-*1,2,3-triazol-4-yl)-2-methylpyridin-3-yl)oxy)octahydropentalene-1-carboxylic acid, and an X-ray powder diffraction pattern expressed in terms of 2*θ* angles, which are diffraction angles, of the crystal form has characteristic peaks at 6.446, 8.350, 8.847, 9.335, 12.845, 16.901, and 17.874.

In some other embodiments, the X-ray powder diffraction pattern expressed in terms of 2*θ* angles, which are diffraction angles, of the crystal form I of the sodium salt has characteristic peaks at 6.446, 8.350, 8.847, 9.335, 9.807, 12.845, 13.328, 13.739, 15.728, 16.901, and 17.874.

In some other embodiments, the X-ray powder diffraction pattern expressed in terms of 2*θ* angles, which are diffraction angles, of the crystal form I of the sodium salt has characteristic peaks at 4.741, 6.446, 8.350, 8.847, 9.335, 9.807, 12.845, 13.328, 13.739, 15.728, 16.901, 17.874, 19.059, and 19.805.

In some other embodiments, the X-ray powder diffraction pattern expressed in terms of 2*θ* angles, which are diffraction angles, of the crystal form I of the sodium salt has characteristic peaks at 4.741, 6.446, 8.350, 8.847, 9.335, 9.807, 12.845, 13.328, 13.739, 15.728, 16.901, 17.874, 19.059, 19.805, 21.490, 23.607, 25.460, and 25.773.

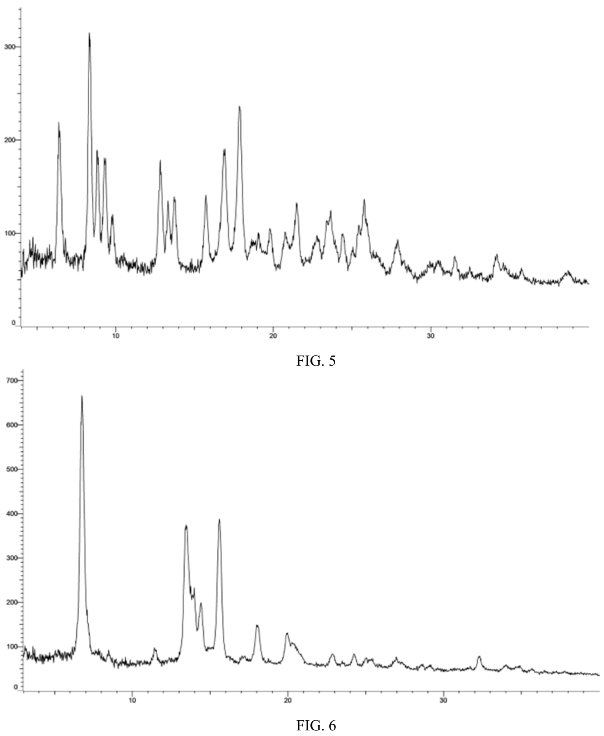

In some other embodiments, the X-ray powder diffraction pattern expressed in terms of 2*θ* angles, which are diffraction angles, of the crystal form I of the sodium salt is shown in FIG. 5.

In another aspect, the present disclosure provides a preparation method for the crystal form I of the sodium salt of the compound of formula I, comprising: dissolving the compound of formula I in 50% ethanol/ethyl acetate, 50% methanol/acetonitrile, or tetrahydrofuran, adding a sodium hydroxide solution, adding methyl tert-butyl ether, and stirring for crystallization.

In another aspect, the present disclosure provides a preparation method for the crystal form I of the sodium salt of the compound of formula I, comprising: dissolving the compound of formula I in 2-butanone or acetonitrile, adding a solution of sodium hydroxide in ethanol, and stirring for crystallization.

In another aspect, the present disclosure further provides a crystal form II of a sodium salt of the compound of formula I, (1S,3aS,5S,6aR)-5-((6-(5-(((4-(methoxymethyl)pyrimidin-2-yl)oxy)methyl)-1-methyl-1*H-*1,2,3-triazol-4-yl)-2-methylpyridin-3-yl)oxy)octahydropentalene-1-carboxylic acid, and an X-ray powder diffraction pattern expressed in terms of 2*θ* angles, which are diffraction angles, of the crystal form has characteristic peaks at 6.771, 13.464, 14.419, and 15.593.

In some other embodiments, the X-ray powder diffraction pattern expressed in terms of 2*θ* angles, which are diffraction angles, of the crystal form II of the sodium salt has characteristic peaks at 6.771, 13.464, 13.970, 14.419, 15.593, 18.044, and 19.952.

In some other embodiments, the X-ray powder diffraction pattern expressed in terms of 2*θ* angles, which are diffraction angles, of the crystal form II of the sodium salt is shown in FIG. 6.

In another aspect, the present disclosure provides a preparation method for the crystal form II of the sodium salt of the compound of formula I, comprising: dissolving the compound of formula I in isopropanol or 1,4-dioxane, adding a solution of sodium hydroxide in ethanol, and stirring for crystallization.

In another aspect, the present disclosure provides a preparation method for the crystal form II of the sodium salt of the compound of formula I, comprising: dissolving the crystal form I of the sodium salt of the compound of formula I in isopropanol or isopropyl acetate and stirring for crystallization.

In another aspect, the present disclosure provides a preparation method for the crystal form II of the sodium salt of the compound of formula I, comprising: dissolving the crystal form I of the sodium salt of the compound of formula I in ethanol or n-propanol, adding n-heptane, and stirring for crystallization.

In another aspect, the present disclosure further provides a crystal form III of a sodium salt of the compound of formula I, (1S,3aS,5S,6aR)-5-((6-(5-(((4-(methoxymethyl)pyrimidin-2-yl)oxy)methyl)-1-methyl-1*H-*1,2,3-triazol-4-yl)-2-methylpyridin-3-yl)oxy)octahydropentalene-1-carboxylic acid, and an X-ray powder diffraction pattern expressed in terms of 2*θ* angles, which are diffraction angles, of the crystal form has characteristic peaks at 6.605, 18.597, 20.094, and 26.882.

In some other embodiments, the X-ray powder diffraction pattern expressed in terms of 2*θ* angles, which are diffraction angles, of the crystal form III of the sodium salt has characteristic peaks at 5.639, 6.605, 10.311, 13.323, 18.597, 20.094, and 26.882.

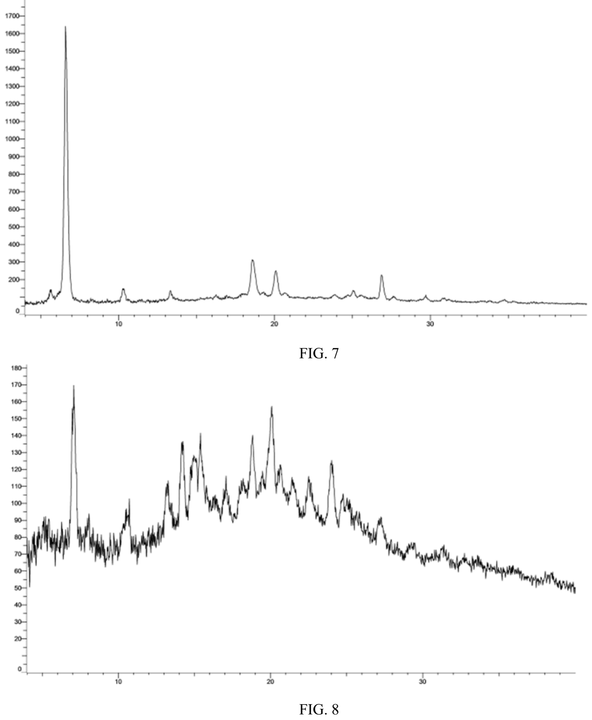

In some other embodiments, the X-ray powder diffraction pattern expressed in terms of 2*θ* angles, which are diffraction angles, of the crystal form III of the sodium salt is shown in FIG. 7.

In another aspect, the present disclosure provides a preparation method for the crystal form III of the sodium salt of the compound of formula I, comprising: dissolving the crystal form I of the sodium salt of the compound of formula I in 1,4-dioxane and stirring for crystallization.

In another aspect, the present disclosure further provides a crystal form IV of a sodium salt of the compound of formula I, (1S,3aS,5S,6aR)-5-((6-(5-(((4-(methoxymethyl)pyrimidin-2-yl)oxy)methyl)-1-methyl-1*H-*1,2,3-triazol-4-yl)-2-methylpyridin-3-yl)oxy)octahydropentalene-1-carboxylic acid, and an X-ray powder diffraction pattern expressed in terms of 2*θ* angles, which are diffraction angles, of the crystal form has characteristic peaks at 7.078, 14.227, 15.004, 18.812, and 20.081.

In some other embodiments, the X-ray powder diffraction pattern expressed in terms of 2*θ* angles, which are diffraction angles, of the crystal form IV of the sodium salt has characteristic peaks at 5.078, 7.078, 10.670, 13.231, 14.227, 15.004, 18.812, 20.081, and 24.018.

In some other embodiments, the X-ray powder diffraction pattern expressed in terms of 2*θ* angles, which are diffraction angles, of the crystal form IV of the sodium salt is shown in FIG. 8.

In another aspect, the present disclosure provides a preparation method for the crystal form IV of the sodium salt of the compound of formula I, comprising: heating the crystal form I of the sodium salt of the compound of formula I to 180 °C or higher.

In another aspect, the present disclosure further provides a crystal form I of an ethanolamine salt of the compound of formula I, (1S,3aS,5S,6aR)-5-((6-(5-(((4-(methoxymethyl)pyrimidin-2-yl)oxy)methyl)-1-methyl-1*H*-1,2,3-triazol-4-yl)-2-methylpyridin-3-yl)oxy)octahydropentalene-1-carboxylic acid, and an X-ray powder diffraction pattern expressed in terms of 2*θ* angles, which are diffraction angles, of the crystal form has characteristic peaks at 5.343, 9.612, 10.751, 16.737, and 19.178.

In some other embodiments, the X-ray powder diffraction pattern expressed in terms of 2*θ* angles, which are diffraction angles, of the crystal form I of the ethanolamine salt has characteristic peaks at 5.343, 9.612, 10.751, 12.023, 14.613, 16.737, 17.886, 19.178, 20.434, and 21.788.

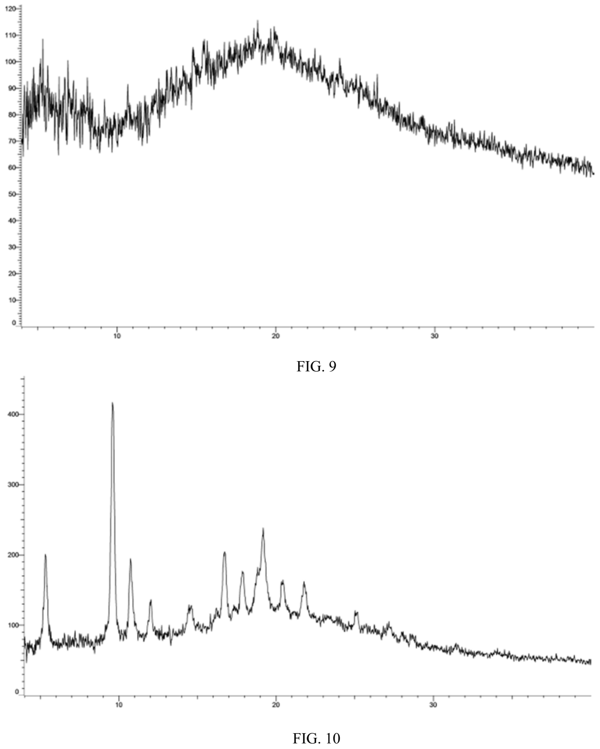

In some other embodiments, the X-ray powder diffraction pattern expressed in terms of 2*θ* angles, which are diffraction angles, of the crystal form I of the ethanolamine salt is shown in FIG. 10.

In another aspect, the present disclosure provides a preparation method for the crystal form I of the ethanolamine salt of the compound of formula I, comprising: dissolving the compound of formula I in tetrahydrofuran, adding a solution of ethanolamine in ethanol, adding methyl tert-butyl ether, and stirring for crystallization.

Further, for the crystal forms of the compound of formula I, (1S,3aS,5S,6aR)-5-((6-(5-(((4-(methoxymethyl)pyrimidin-2-yl)oxy)methyl)-1-methyl-1*H*-1,2,3-triazol-4-yl)-2-methylpyridin-3-yl)oxy)octahydropentalene-1-carboxylic acid, of the present disclosure, the 2*θ* angles have a margin of error of ±0.2.

In certain embodiments, the preparation methods for the crystal forms described in the present disclosure further comprise a crystallization, filtration, washing, or drying step.

In another aspect, the present disclosure further provides a pharmaceutical composition comprising the aforementioned crystal form A, crystal form B, crystal form C, amorphous form, sodium salt, crystal form I of the sodium salt, crystal form II of the sodium salt, crystal form III of the sodium salt, crystal form IV of the sodium salt, amorphous form of the sodium salt, ethanolamine salt, or crystal form I of the ethanolamine salt of the compound of formula I, (1S,3aS,5S,6aR)-5-((6-(5-(((4-(methoxymethyl)pyrimidin-2-yl)oxy)methyl)-1-methyl-1H-1,2,3-triazol-4-yl)-2-methylpyridin-3-yl)oxy)octahydropentalene-1-carboxylic acid, and optionally a pharmaceutically acceptable excipient.

The present disclosure further provides a preparation method for a pharmaceutical composition, comprising a step of mixing the aforementioned crystal form A, crystal form B, crystal form C, amorphous form, sodium salt, crystal form I of the sodium salt, crystal form II of the sodium salt, crystal form III of the sodium salt, crystal form IV of the sodium salt, amorphous form of the sodium salt, ethanolamine salt, or crystal form I of the ethanolamine salt of the compound of formula I, (1S,3aS,5S,6aR)-5-((6-(5-(((4-(methoxymethyl)pyrimidin-2-yl)oxy)methyl)-1-methyl-1H-1,2,3-triazol-4-yl)-2-methylpyridin-3-yl)oxy)octahydropentalene-1-carboxylic acid, with a pharmaceutically acceptable excipient.

The present disclosure further provides use of the aforementioned crystal form A, crystal form B, crystal form C, amorphous form, sodium salt, crystal form I of the sodium salt, crystal form II of the sodium salt, crystal form III of the sodium salt, crystal form IV of the sodium salt, amorphous form of the sodium salt, ethanolamine salt, or crystal form I of the ethanolamine salt of the compound of formula I, (1S,3aS,5S,6aR)-5-((6-(5-(((4-(methoxymethyl)pyrimidin-2-yl)oxy)methyl)-1-methyl-1H-1,2,3-triazol-4-yl)-2-methylpyridin-3-yl)oxy)octahydropentalene-1-carboxylic acid, or the aforementioned pharmaceutical composition as an LPA1 inhibitor.

The present disclosure further provides use of the aforementioned crystal form A, crystal form B, crystal form C, amorphous form, sodium salt, crystal form I of the sodium salt, crystal form II of the sodium salt, crystal form III of the sodium salt, crystal form IV of the sodium salt, amorphous form of the sodium salt, ethanolamine salt, or crystal form I of the ethanolamine salt of the compound of formula I, (1S,3aS,5S,6aR)-5-((6-(5-(((4-(methoxymethyl)pyrimidin-2-yl)oxy)methyl)-1-methyl-1H-1,2,3-triazol-4-yl)-2-methylpyridin-3-yl)oxy)octahydropentalene-1-carboxylic acid, or the aforementioned pharmaceutical composition for preventing and/or treating fibrotic diseases of organs, respiratory diseases, renal diseases, hepatic diseases, inflammatory diseases, neurological diseases, cardiovascular and cerebrovascular diseases, gastrointestinal diseases, pain, urological diseases, ophthalmic diseases, metabolic diseases, cancer, and transplant rejection. The present disclosure further provides use of the aforementioned crystal form A, crystal form B, crystal form C, amorphous form, sodium salt, crystal form I of the sodium salt, crystal form II of the sodium salt, crystal form III of the sodium salt, crystal form IV of the sodium salt, amorphous form of the sodium salt, ethanolamine salt, or crystal form I of the ethanolamine salt of the compound of formula I, (1S,3aS,5S,6aR)-5-((6-(5-(((4-(methoxymethyl)pyrimidin-2-yl)oxy)methyl)-1-methyl-1H-1,2,3-triazol-4-yl)-2-methylpyridin-3-yl)oxy)octahydropentalene-1-carboxylic acid, or the aforementioned pharmaceutical composition in the manufacture of a medicament for preventing and/or treating fibrotic diseases of organs, respiratory diseases, renal diseases, hepatic diseases, inflammatory diseases, neurological diseases, cardiovascular and cerebrovascular diseases, gastrointestinal diseases, pain, urological diseases, ophthalmic diseases, metabolic diseases, cancer, and transplant rejection.

As used herein, "2*θ* or 2*θ* angle" refers to a diffraction angle; *θ* refers to the Bragg angle in ° or degrees; the 2*θ* of each characteristic peak has a margin of error of ±0.20 (including the case that a number having more than 1 decimal place is rounded), specifically -0.20, -0.19, -0.18, -0.17, -0.16, -0.15, -0.14, -0.13, -0.12, - 0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

In the present disclosure, there is a certain degree of error in the measurement of the chemical ratio of the compound to the acid molecule. Generally, ±10% is considered a reasonable margin of error. The error varies to some extent depending on the context in which it is used, and the variation does not exceed ±10% and may be ±9%, ±8%, ±7%, ±6%, ±5%, ±4%, ±3%, ±2%, or ±1%, preferably ±5%. The numerical values described with "about" in the present disclosure have the aforementioned reasonable margin of error.

As used herein, "crystallization" or "crystallizing" includes, but is not limited to, stirring crystallization, slurrying crystallization, cooling crystallization, and volatilizing crystallization.

As used herein, "differential scanning calorimetry" or "DSC" refers to the measurement of the temperature difference and heat flow difference between a sample and a reference substance during a ramping or thermostatic process to characterize all the physical changes and chemical changes related to the thermal effect, and to obtain phase change information about the sample.

In the present disclosure, the drying temperature is generally 25 °C-100 °C, preferably 40 °C-70 °C, and the drying may be performed under atmospheric pressure or reduced pressure.

As used herein, "pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

### BRIEF DESCRIPTION OF THE DRAWINGS

FIG. 1 shows an XRPD pattern of the amorphous form of the compound of formula I.
FIG. 2 shows an XRPD pattern of the crystal form A of the compound of formula I.
FIG. 3 shows an XRPD pattern of the crystal form B of the compound of formula I.
FIG. 4 shows an XRPD pattern of the crystal form C of the compound of formula I.
FIG. 5 shows an XRPD pattern of the crystal form I of the sodium salt of the compound of formula I.
FIG. 6 shows an XRPD pattern of the crystal form II of the sodium salt of the compound of formula I.
FIG. 7 shows an XRPD pattern of the crystal form III of the sodium salt of the compound of formula I.
FIG. 8 shows an XRPD pattern of the crystal form IV of the sodium salt of the compound of formula I.
FIG. 9 shows an XRPD pattern of the amorphous form of the sodium salt of the compound of formula I.
FIG. 10 shows an XRPD pattern of the crystal form I of the ethanolamine salt of the compound of formula I.

### DETAILED DESCRIPTION

The present disclosure is further illustrated in detail by the following examples and experimental examples. These examples and experimental examples are illustrative only and are not intended to limit the scope of the present disclosure.

Test conditions for the instruments used in the experiments:
The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument, with dimethyl sulfoxide-D6 (DMSO-*d*₆), chloroform-D (CDCl₃), and methanol-D4 (CD₃OD) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), waters ACQuity UPLC-QD/SQD (manufacturer: waters; MS model: waters ACQuity Qda Detector/waters SQ Detector), and THERMO Ultimate 3000-Q Exactive (manufacturer: THERMO; MS model: THERMO Q 15 Exactive). The HPLC analyses were performed using an Agilent 1260DAD high pressure liquid chromatograph (Sunfire C18 150 × 4.6 mm chromatography column) and a Thermo U3000 high pressure liquid chromatograph (Gimini C18 150 × 4.6 mm chromatography column). XRPD refers to X-ray powder diffraction: The analysis was performed using a BRUKER D8 X-ray diffractometer; specific acquisition information: a Cu anode (40 kV, 40 mA), ray: monochromatic Cu-Ka ray (1 = 1.5418 Å). Scan mode: *θ*/2*θ*; scan range: 3-48°. DSC refers to differential scanning calorimetry: The analysis was performed using a METTLER TOLEDO DSC 3+ differential scanning calorimeter, with a temperature ramp rate of 10 °C/min, specific temperature ranges shown in corresponding patterns (mostly 25 °C-300 °C), and a nitrogen purge speed of 50 mL/min. TGA refers to thermogravimetric analysis: The analysis was performed using a METTLER TOLEDO TGA 2 thermogravimetric analyzer, with a temperature ramp rate of 10 °C/min, specific temperature ranges shown in corresponding patterns (mostly 30 °C-400 °C), and a nitrogen purge speed of 50 mL/min. DVS refers to dynamic vapor sorption: Surface Measurement Systems instrinsic was adopted; the humidity started at 50%, the humidity range of the investigation was 0%-95%, and the humidity was increased in increments of 10%; the criterion was as follows: each gradient mass change dM/dT ≤ 0.002%; TMAX 360 min, two cycles.

The known starting materials in the present disclosure may be synthesized by using or according to methods known in the art, or may be purchased from companies such as ABCR GmbH & Co. KG, Acros Organics, Aldrich Chemical Company, Accela ChemBio Inc., and Chembee Chemicals.

The monitoring of the reaction progress in the examples was performed by thin-layer chromatography (TLC). The developing solvents for the reactions, the eluent systems for the column chromatography purification, and the developing solvent systems for the thin-layer chromatography include: A: a dichloromethane/methanol system, and B: a n-hexane/ethyl acetate system. The volume ratio of the solvents was adjusted depending on the polarity of the compound, or small amounts of basic or acidic reagents such as triethylamine and acetic acid may also be added for adjustments.

### Example 1: Preparation of Compound I (compound 1-P1, with reference to the preparation method of Example 3 in Application No. PCT/CN2022/106706)

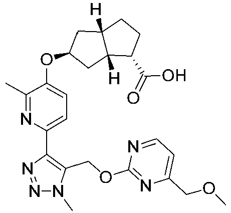

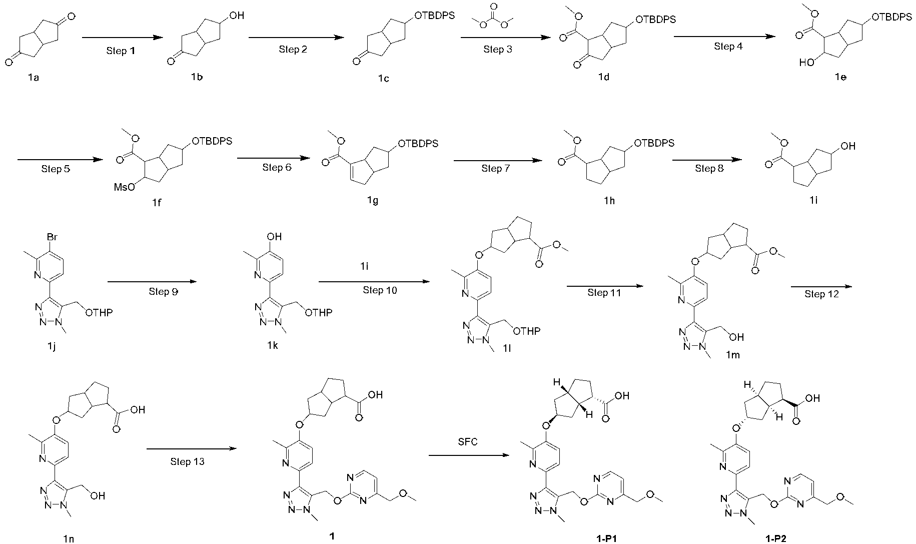

### Step 1: 5-hydroxyhexahydropentalen-2(1H)-one (1b)

The starting material cis-tetrahydropentalene-2,5(1*H*,3*H*)-dione **1a** (24 g, 173.7 mmol) was dissolved in ethanol (500 mL), and the solution was cooled to 0 °C. Sodium borohydride (1.47 g, 43.4 mmol) was added, and the mixture was stirred at 0 °C for 2 h. The reaction mixture was quenched with acetic acid (10 mL) and concentrated, and the residue was purified by silica gel flash column chromatography (petroleum ether/ethyl acetate) to give the title product **1b** (9 g, 37.0% yield). ¹H NMR: (400 MHz, CDCl₃) δ 4.55-4.37 (m, 1H), 2.90-2.75 (m, 2H), 2.64-2.48 (m, 2H), 2.36-2.14 (m, 4H), 1.65-1.49 (m, 3H).

### Step 2: 5-((tert-butyldiphenylsilyl)oxy)hexahydropentalen-2(1H)-one (1c)

**1b** (15 g, 107.0 mmol) was dissolved in dichloromethane (150 mL), and imidazole (21.8 g, 321.0 mmol) was added. After the mixture was cooled to 0 °C, tert-butyl(chloro)diphenylsilane (30.5 mL, 117.7 mmol) was added, and the mixture was stirred at 0 °C for 1 h. The reaction mixture was washed with a 1 M hydrochloric acid solution (150 mL × 3) and saturated brine (300 mL), dried over anhydrous sodium sulfate, and filtered. The filtrate was concentrated, and the residue was purified by silica gel flash column chromatography (petroleum ether/ethyl acetate) to give the title product **1c** (41 g, crude). The product was directly used in the next step without further purification.

### Step 3: methyl 5-((tert-butyldiphenylsilyl)oxy)-2-oxooctahydropentalene-1-carboxylate (1d)

A solution of **1c** (41 g, 108.3 mmol) in tetrahydrofuran (500 mL) was cooled to 0 °C, and after 60% sodium hydride (17.3 g, 433.2 mmol) was added, dimethyl carbonate (19.5 g, 216.6 mmol) was added. The mixture was heated to 60 °C and stirred for 3 h. Water (150 mL) was added to quench the reaction. The reaction mixture was extracted with ethyl acetate (100 mL × 3). The organic phases were combined, washed with saturated brine (300 mL), dried over anhydrous sodium sulfate, and filtered. The filtrate was concentrated, and the residue was purified by silica gel flash column chromatography (petroleum ether/ethyl acetate) to give the title product **1d** (25 g, 52.9% yield). MS (ESI): m/z = 459.1 [M+Na]⁺.

### Step 4: methyl 5-((tert-butyldiphenylsilyl)oxy)-2-hydroxyoctahydropentalene-1-carboxylate (1e)

**1d** (32 g, 73.3 mmol) was dissolved in tetrahydrofuran (350 mL), and the solution was cooled to 0 °C. Sodium borohydride (2.48 g, 73.3 mmol) was added, and the mixture was stirred at 0 °C for 3 h. The reaction mixture was quenched with a 0.5 M hydrochloric acid solution (30 mL) and extracted with ethyl acetate (30 mL × 3). The organic phases were combined, washed with saturated brine (30 mL), dried over anhydrous sodium sulfate, and filtered. The filtrate was concentrated, and the residue was purified by silica gel flash column chromatography (petroleum ether/ethyl acetate) to give the title product **1e** (9 g, 28.0% yield). MS (ESI): m/z = 461.2 [M+Na]⁺.

### Step 5: methyl 5-((tert-butyldiphenylsilyl)oxy)-2-((methanesulfonyl)oxy)octahydropentalene-1-carboxylate (1f)

**1e** (9 g, 20.5 mmol) was dissolved in dichloromethane (20 mL), and triethylamine (5.19 g, 51.3 mmol) was added. The mixture was cooled to 0 °C, and after methanesulfonic anhydride (5.36 g, 30.8 mmol) was added, the reaction mixture was warmed to room temperature and stirred for 3 h. The reaction mixture was washed with water (25 mL × 2) and saturated brine (30 mL), dried over anhydrous sodium sulfate, and filtered. The filtrate was concentrated, and the residue was purified by silica gel flash column chromatography (petroleum ether/ethyl acetate) to give the title product **1f** (9 g, 84.9% yield). MS (ESI): m/z = 539.1 [M+Na]⁺.

### Step 6: methyl 5-((tert-butyldiphenylsilyl)oxy)-3,3a,4,5,6,6a-hexahydropentalene-1-carboxylate (1g)

**1f** (9 g, 17.4 mmol) was dissolved in toluene (100 mL), and 1,8-diazabicyclo[5.4.0]undec-7-ene (5.3 g, 34.8 mmol) was added. The mixture was heated to 90 °C and stirred for 2 h. The reaction mixture was concentrated, and the residue was purified by silica gel flash column chromatography (petroleum ether/ethyl acetate) to give the title product **1g** (6.5 g, 88.7% yield). MS (ESI): m/z = 443.2 [M+Na]⁺.

### Step 7: methyl 5-((tert-butyldiphenylsilyl)oxy)octahydropentalene-1-carboxylate (1h)

**1g** (6.5 g, 15.5 mmol) was dissolved in methanol (65 mL), and 10% Pd/C (600 mg) was added in a nitrogen atmosphere. After the mixture was purged with hydrogen several times, the reaction mixture was stirred at room temperature for 3 h in a hydrogen atmosphere (15 psi). The reaction mixture was filtered, and the filtrate was concentrated to give the title product **1h** (crude, 6.5 g). The product was directly used in the next step. MS (ESI): m/z = 445.2 [M+Na]⁺.

### Step 8: methyl 5-hydroxyoctahydropentalene-1-carboxylate (1i)

**1h** (6.3 g, 14.9 mmol) was dissolved in tetrahydrofuran (60 mL), and a 1 M solution of tetrabutylammonium fluoride in tetrahydrofuran (16.4 mL, 16.4 mmol) was added. The mixture was left to react at room temperature for 3 h. Water (30 mL) was added to the reaction mixture, and extraction was performed with ethyl acetate (20 mL × 3). The organic phases were combined, washed with saturated brine (60 mL), dried over anhydrous sodium sulfate, and filtered. The filtrate was concentrated, and the residue was purified by silica gel flash column chromatography (petroleum ether/ethyl acetate) to give the title product **1i** (2.1 g, 76.5% yield). ¹H NMR (400 MHz, CDCl₃) δ 4.03 (tt, *J =* 9.8, 6.1 Hz, 1H), 3.65 (s, 3H), 2.75-2.61 (m, 2H), 2.50-2.38 (m, 1H), 2.26-2.15 (m, 1H), 2.02-1.88 (m, 2H), 1.83-1.74 (m, 2H), 1.66-1.49 (m, 2H), 1.22-1.07 (m, 2H).

### Step 9: 2-methyl-6-(1-methyl-5-(((tetrahydro-2H-pyran-2-yl)oxy)methyl)-1H-1,2,3-triazol-4-yl)pyridin-3-ol (1k)

3-Bromo-2-methyl-6-(1-methyl-5-((tetrahydro-2*H*-pyran-2-yl)oxy)methyl)-1*H*-1,2,3-triazol-4-yl)pyridine **1j** (2.5 g, 6.81 mmol, prepared using the method disclosed in Example 1 in the specification of the patent application WO2017223016A1), bis(dibenzylideneacetone)palladium (391 mg, 0.68 mmol), 2-di-tert-butylphosphino-2',4',6'-triisopropylbiphenyl (578 mg, 1.36 mmol), and potassium hydroxide (1.15 g, 20.44 mmol) were dissolved in 1,4-dioxane (50 mL) and water (10 mL), and the mixture was left to react at 100 °C for 2 h in a nitrogen atmosphere. The pH was adjusted to 6-7 with a 1 M hydrochloric acid solution, and water (10 mL) was added. Extraction was performed with ethyl acetate (50 mL × 2), and the organic phases were combined, washed with saturated brine, dried over anhydrous sodium sulfate, filtered, and concentrated to give a crude product. The crude product was purified by silica gel flash column chromatography (ethyl acetate/petroleum ether) to give the title product **1k** (1.9 g, 92% yield). MS (ESI) m/z = 305.6 [M+H]⁺.

### Step 10: methyl 5-((2-methyl-6-(1-methyl-5-(((tetrahydro-2H-pyran-2-yl)oxy)methyl)-1H-1,2,3-triazol-4-yl)pyridin-3-yl)oxy)octahydropentalene-1-carboxylate (1l)

**1k** (1 g, 3.29 mmol), triphenylphosphine (2.59 g, 9.86 mmol), and **1i** (605 mg, 3.29 mmol) were dissolved in tetrahydrofuran (50 mL), and the solution was heated to 50 °C. A solution of di-tert-butyl azodicarboxylate (2.25 g, 9.86 mmol) in tetrahydrofuran (10 mL) was slowly added dropwise, and the mixture was stirred at 50 °C for 2 h. The reaction mixture was concentrated under reduced pressure to give a crude product, and the crude product was purified through a reversed-phase C18 column (acetonitrile/water) to give the title product **1l** (1.2 g, 77% yield). MS (ESI) m/z = 471.8 [M+H]⁺.

### Step 11: methyl 5-((6-(5-(hydroxymethyl)-1-methyl-1H-1,2,3-triazol-4-yl)-2-methylpyridin-3-yl)oxy) octahydropentalene-1-carboxylate (1m)

**1l** (1.2 g, 2.55 mmol) was dissolved in methanol (30 mL), and p-toluenesulfonic acid hydrate (194 mg, 1.02 mmol) was added. The reaction mixture was heated to 70 °C and left to react for 2 h in a nitrogen atmosphere. The reaction mixture was cooled and concentrated under reduced pressure to give the title product **1m** (crude, 900 mg). The product was directly used in the next step. MS (ESI) m/z = 387.7 [M+H]⁺.

### Step 12: 5-((6-(5-(hydroxymethyl)-1-methyl-1H-1,2,3-triazol-4-yl)-2-methylpyridin-3-yl)oxy)octahydropentalene-1-carboxylic acid (1n)

**1m** (900 mg, 2.33 mmol) was dissolved in tetrahydrofuran (10 mL), methanol (10 mL), and water (10 mL), and lithium hydroxide (536 mg, 23.29 mmol) was then added. The mixture was left to react at room temperature for 3 h. The pH was adjusted to 5-6 with a 1 M hydrochloric acid solution, and water (10 mL) was added. Extraction was performed with ethyl acetate (30 mL × 2), and the organic phases were combined, washed with saturated brine, dried over anhydrous sodium sulfate, and filtered. The filtrate was concentrated to give the title product **1n** (800 mg, 82% yield). MS (ESI) m/z = 373.6 [M+H]⁺.

### Step 13: (1S,3aS,5S,6aR)-5-((6-(5-(((4-(methoxymethyl)pyrimidin-2-yl)oxy)methyl)-1-methyl-1H-1,2,3-triazol-4-yl)-2-methylpyridin-3-yl)oxy)octahydropentalene-1-carboxylic acid (1)

**1n** (350 mg, 0.94 mmol) was dissolved in N,N-dimethylacetamide (20 mL), and 60% sodium hydride (180 mg, 7.52 mmol) was added under ice bath conditions. After 30 min of reaction, 2-chloro-4-(methoxymethyl)pyrimidine (179 mg, 1.13 mmol) was added, and the mixture was left to react at room temperature for 30 min. The reaction mixture was diluted with water (10 mL), and the pH was adjusted to 4-5 with 1 M hydrochloric acid. Extraction was performed with ethyl acetate (20 mL × 2). The organic phases were combined, washed with saturated brine, dried over anhydrous sodium sulfate, and filtered. The filtrate was concentrated to give a crude product, and the crude product was purified by reversed-phase flash column chromatography (acetonitrile/water) followed by lyophilization to give compound **1** (200 mg, 43% yield). MS (ESI) m/z = 495.5 [M+H]⁺

Compound **1** was resolved by preparative chiral SFC (resolution conditions: preparative chiral SFC column DAICEL CHIRALPAK AS (250 mm × 30 mm, 10 µm); mobile phases: A was CO₂, and B was ethanol (0.1% ammonia water)). The corresponding fractions were collected and concentrated under reduced pressure to give compounds **1-P1** and **1-P2.**

Compound **1-P1** was the one with a shorter retention time: MS m/z (ESI): 495.2 [M+H]⁺; chiral SFC analysis: retention time 2.717 min, chiral purity 100% (column: Chiralpak AS-3, 0.46 cm I.D. × 100 mm, 3 µm; mobile phases: A was CO₂, and B was ethanol (0.05% diethylamine). ¹H NMR (400 MHz, CDCl₃) δ 8.50 (d, *J* = 5.0 Hz, 1H), 7.90 (d, *J* = 8.5 Hz, 1H), 7.13 (d, *J =* 6.6 Hz, 2H), 6.16-6.01 (m, 2H), 4.86 (s, 1H), 4.36 (s, 2H), 4.18 (s, 3H), 3.45 (s, 3H), 3.15-2.98 (m, 1H), 2.91-2.77 (m, 2H), 2.34 (s, 3H), 2.30-2.21 (m, 1H), 2.09-2.00 (m, 1H), 1.87-1.77 (m, 2H), 1.75-1.64 (m, 1H), 1.57-1.40 (m, 3H).

Compound **1-P2** was the one with a longer retention time: MS m/z (ESI): 495.2 [M+H]⁺; chiral SFC analysis: retention time 3.031 min, chiral purity 97.4% (column: Chiralpak AS-3, 0.46 cm I.D. × 100 mm, 3 µm; mobile phases: A was CO₂, and B was ethanol (0.05% diethylamine). ¹H NMR (400 MHz, CDCl₃) δ 8.50 (d, *J* = 5.0 Hz, 1H), 7.91 (d, *J =* 8.5 Hz, 1H), 7.16-7.10 (m, 2H), 6.17-6.01 (m, 2H), 4.87 (s, 1H), 4.36 (s, 2H), 4.18 (s, 3H), 3.45 (s, 3H), 3.14-3.00 (m, 1H), 2.91-2.78 (m, 2H), 2.35 (s, 3H), 2.31-2.21 (m, 1H), 2.11-2.02 (m, 1H), 1.89-1.78 (m, 2H), 1.75-1.63 (m, 1H), 1.58-1.39 (m, 3H).

The product compound **1-P1** was identified as an amorphous form by X-ray powder diffraction. Its XRPD pattern is shown in FIG. 1.

### Example 2: Antagonist Property Test of Compound

The antagonist properties of a compound of the present disclosure were assessed using the FLIPR (fluorometric imaging plate reader) method. The compound is an inhibitor of intracellular calcium elevation induced by activation of hLPAR1 (human lysophosphatidic acid receptor 1) expressed in CHO-K1 cells (Chinese hamster ovary K1 cells, HDB).
1. Experimental reagents and instrument consumables

| Reagents and instrument consumables | Supplier | Cat. No. |
|---|---|---|
| CHO-K1 cells | HDB | |
| Nutrient mixture F-12 Ham | Gibco | 21700 |
| Fetal bovine serum (FBS) | biosera | FB-1058/500 |
| Hygromycin B | CALBIOCHEM | 400052-20 mL |
| 0.25% trypsin | Invitrogen | 25200 |
| Calcium ion fluorescent probe Fluo-8 | AAT Bioquest | 21080 |
| Probenecid | Invitrogen | P36400 |
| Tartrazine | Sigma | T0388-100G |
| Acid red | ALDRICH | 210634-25G |
| HBSS (containing Ca/Mg) | Sigma | H1387 |
| HEPES, pH 7.4 | gibco | 15630-080 |
| Bovine serum albumin, free of fatty acids | Proliant | 69700 |
| FLIPR | Molecular Devices | HD-4HYSG2600 |
| Oleoyl-L-α-lysophosphatidic acid sodium salt (LPA) | Sigma | L7260 |

2. Reagent preparation
2.1. Reaction buffer: HBSS + 20 mM HEPES + 0.1% fatty acid-free BSA + 0.001% F-127
2.2. 50× Red dye: (a red dye for blocking background signals in cells) prepared by: weighing out 4 g of tartrazine and 10.2 g of acid red and dissolving them in 100 mL of H₂O.
2.3. Fluo-8 staining mixture: 4 mL of reaction buffer, 32 µL of fluo-8, 320 µL of 50× Red dye, and 40 µL of probenecid.

3. Compound preparation
3.1. LPA: It was dissolved in DPBS (containing 0.1% fatty acid-free BSA) to prepare a 0.8 µM stock solution, and the solution was aliquoted and stored at -20 °C.
3.2. Test compound preparation: The test compound was dissolved in DMSO to prepare a 10 mM stock solution, and the solution was stored at -20 °C. In the experiment, the compound was first gradient-diluted with DMSO (starting concentration: 50 µM, 3-fold dilution, 10 points) to prepare a 200× compound solution. The compound solution was then diluted with reaction buffer to form a 5× compound solution, and the solution was transferred to a 384-well plate (Cat. No. 6008590) using Bravo.

4. Experimental steps
4.1. CHO-Kl/LPA1R cells were cultured in a cell culture medium (F-12 + 10% FBS + 400 µg/mL hygromycin B).
4.2. When the cell confluence reached 80%, the cells were digested with 0.25% trypsin.
4.3. When the cells became round, the digestion was stopped with culture medium F-12 (10% FBS), and the cells were counted. Then the cells were diluted in F-12 (10% FBS) to form a cell suspension with a density of 6.7 × 10⁵ cells/mL.
4.4. The cell suspension was added to a 384-well black cell culture plate at 30 µL/well using a Multidrop auto-dispenser, and the plate was incubated in a 5% CO₂ incubator at 37 °C for 20-24 h. The culture medium was changed to a serum-free F-12 culture medium, and the cells were starved for 24 h.
4.5. Dose experiment for testing LPA. 6× LPA solution gradient dilutions (starting concentration: 60 µM, 3-fold dilution, 10 points) were prepared with reaction buffer. The culture medium in the 384-well black cell culture plate was discarded and changed to the reaction buffer. 10 µL of a 5% DMSO reaction buffer was added, and 10 µL of the Fluo-8 staining mixture was then added. The plate was incubated in the dark in a 5% CO₂ incubator at 37 °C for 0.5 h.
4.6. An FLIPR was correspondingly programmed, the 6× LPA gradient solutions were added, and readings were taken. Two-minute data were collected.
4.7. A reaction curve was obtained from the numerical values of LPA, and the concentration of LPA at EC₈₀ was calculated. A 6× LPA solution was prepared in a 384-well plate (Cat. No. 6008590), and the HPE and ZPE were added to corresponding wells. The HPE (one hundred per cent effect) was 60 µM LPA, and the ZPE (zero per cent effect) was reaction buffer.
4.8. Compound test. The culture medium in the 384-well black cell culture plate was discarded and changed to the reaction buffer. 10 µL of the 5× compound solution was transferred using Bravo, and 10 µL of the Fluo-8 staining mixture was then added immediately. The plate was incubated in the dark in a 5% CO₂ incubator at 37 °C for 0.5 h.
4.9. An FLIPR was correspondingly programmed, the 6× LPA solution was added, and readings were taken. Two-minute data were collected.
4.10. Finally, the output fluorescence counts were analyzed, and the IC50 of the compound was calculated.

5. Experimental results

**Table 1. The IC₅₀ of the compound of the present disclosure obtained against the LPAR1 receptor**

| Compound | LPAR1 IC₅₀ (nM) |
|---|---|
| **1-P1** | 8.7 |

Conclusion: The results show that the above compound has excellent inhibitory activity against LPAR1, and as an LPAR1 antagonist, it can be applied in the treatment of diseases related to the LPAR1 target.

### Example 3: Preparation of Crystal Form A of Compound of Formula I

About 300 mg of the compound represented by formula I was weighed out and dissolved in 7 mL of propylene glycol methyl ether, and 21 mL of n-heptane was added. The reaction mixture was stirred for 1 day, and the solid was collected by filtration under vacuum and dried to give the crystal form A of the compound of formula I.

X-ray powder diffraction analysis was performed. The XRPD pattern is shown in FIG. 2, and its characteristic peak positions are shown in Table 2. The TGA thermogram showed a weight loss of 1.55% before 100 °C. The DSC thermogram showed an endothermic peak at 183.35 °C.

DVS test results showed that the sample had a hygroscopic weight gain of about 0.17% under normal storage conditions (i.e., 25 °C and 60% RH), a hygroscopic weight gain of about 0.20% under accelerated experimental conditions (i.e., 70% RH), and a hygroscopic weight gain of about 0.37% under extreme conditions (90% RH). After the DVS test, the sample was re-identified, and the results showed that its crystal form did not change.

**Table 2**

| Peak No. | 2θ value [° or degrees] | d[Å] | Relative intensity% |
|---|---|---|---|
| 1 | 7.534 | 11.72515 | 33.6 |
| 2 | 10.223 | 8.64609 | 100.0 |
| 3 | 11.041 | 8.00708 | 6.6 |
| 4 | 12.759 | 6.93245 | 6.5 |
| 5 | 13.421 | 6.59213 | 8.2 |
| 6 | 14.364 | 6.16145 | 43.4 |
| 7 | 15.008 | 5.89850 | 9.1 |
| 8 | 15.816 | 5.59895 | 15.2 |
| 9 | 16.678 | 5.31118 | 25.5 |
| 10 | 17.269 | 5.13100 | 40.1 |
| 11 | 18.023 | 4.91794 | 13.1 |
| 12 | 18.495 | 4.79342 | 42.6 |
| 13 | 19.553 | 4.53628 | 40.0 |
| 14 | 20.413 | 4.34715 | 11.2 |
| 15 | 21.043 | 4.21842 | 24.2 |
| 16 | 21.342 | 4.15995 | 32.3 |
| 17 | 22.293 | 3.98458 | 6.6 |
| 18 | 23.074 | 3.85150 | 12.9 |
| 19 | 23.647 | 3.75939 | 16.7 |
| 20 | 24.382 | 3.64775 | 2.9 |
| 21 | 24.820 | 3.58437 | 5.9 |
| 22 | 25.359 | 3.50939 | 4.2 |
| 23 | 25.965 | 3.42879 | 34.4 |
| 24 | 28.461 | 3.13356 | 8.1 |
| 25 | 30.356 | 2.94209 | 11.2 |
| 26 | 34.118 | 2.62581 | 6.8 |

### Example 4: Preparation of Crystal Form A of Compound of Formula I

About 5 mg of the compound represented by formula (I) was weighed out and completely dissolved in solvent A. Solvent B was added, and solvent C was then added. The mixture was stirred for 3 days, and a solid precipitated. The solid was collected by centrifugation and dried to give the crystal form A of the compound of formula (I).

**Table 3. Preparation of the crystal form A of the compound of formula I**

| NO | Formula I | Solvent A | Solvent B | Solvent C | Procedure |
|---|---|---|---|---|---|
| 1 | 5 mg | 0.25 mL of propylene glycol methyl ether | 0.2 mL of methyl tert-butyl ether | 0.4 mL of n-heptane | Stirring for 3 days |
| 2 | | 0.25 mL of 1,4-dioxane | | | |
| 3 | | 0.25 mL of 10% water/acetone | | | |
| 4 | 5 mg | 0.05 mL ofN,N-dimethylformamide | 0.2 mL of methyl tert-butyl | 0.2 mL of n-heptane | Stirring for 1 day |
| 5 | | 0.05 mL of N,N-dimethylacetamide | ether | | |
| 6 | | 0.05 mL of 50% methanol/chloroform | | | |
| 7 | | 0.05 mL of 67% tetrahydrofuran/ethanol | | | |
| 8 | 5 mg | 0.25 mL of acetone | \ | \ | Stirring for 3 days |

### Example 5: Preparation of Crystal Form B of Compound of Formula I

About 5 mg of the compound represented by formula (I) was weighed out and completely dissolved in 0.1 mL of tetrahydrofuran (solvent A). 0.2 mL of methyl tert-butyl ether (solvent B) was added, and 0.2 mL of n-heptane (solvent C) was then added. The mixture was stirred for 1 day, and a solid precipitated. The solid was collected by centrifugation and dried to give the crystal form B of the compound of formula I.

**Table 4. Preparation of the crystal form B of the compound of formula I**

| NO | Formula I | Solvent A | Solvent B | Solvent C |
|---|---|---|---|---|
| 1 | 5 mg | 0.1 mL of dichloromethane | 0.2 mL of methyl tert-butyl ether | 0.2 mL of n-heptane |
| 2 | | 0.1 mL of 10% water/isopropanol | | |
| 3 | | 0.1 mL of trichloromethane | | |

X-ray powder diffraction analysis was performed. The XRPD pattern is shown in FIG. 3, and its characteristic peak positions are shown in Table 5. The TGA thermogram showed a weight loss of 1.66% before 100 °C. The DSC thermogram showed endothermic peaks at 163.19 °C and 182.43 °C and an exothermic peak at 164.04 °C.

DVS test results showed that the sample had a hygroscopic weight gain of about 0.25% under normal storage conditions (i.e., 25 °C and 60% RH), a hygroscopic weight gain of about 0.31% under accelerated experimental conditions (i.e., 70% RH), and a hygroscopic weight gain of about 0.64% under extreme conditions (90% RH). After the DVS test, the sample was re-identified, and the results showed that its crystal form did not change.

**Table 5**

| Peak No. | 2θ value [° or degrees] | d[Å] | Relative intensity% |
|---|---|---|---|
| 1 | 5.055 | 17.46698 | 6.4 |
| 2 | 7.437 | 11.87807 | 93.8 |
| 3 | 10.381 | 8.51471 | 14.9 |
| 4 | 14.717 | 6.01432 | 49.4 |
| 5 | 14.955 | 5.91922 | 100.0 |
| 6 | 15.993 | 5.53712 | 1.8 |
| 7 | 16.970 | 5.22048 | 2.6 |
| 8 | 17.513 | 5.06004 | 26.2 |
| 9 | 18.466 | 4.80089 | 11.8 |
| 10 | 21.395 | 4.14984 | 15.8 |
| 11 | 21.692 | 4.09356 | 11.9 |
| 12 | 22.607 | 3.92994 | 17.5 |
| 13 | 23.221 | 3.82748 | 16.2 |
| 14 | 24.716 | 3.59913 | 10.3 |
| 15 | 25.972 | 3.42786 | 11.9 |
| 16 | 26.302 | 3.38563 | 3.4 |
| 17 | 27.307 | 3.26332 | 3.7 |
| 18 | 28.840 | 3.09324 | 5.9 |
| 19 | 29.426 | 3.03295 | 8.1 |
| 20 | 31.659 | 2.82395 | 1.3 |
| 21 | 32.397 | 2.76130 | 4.2 |
| 22 | 33.276 | 2.69032 | 0.6 |

### Example 6: Preparation of Crystal Form B of Compound of Formula I

About 5 mg of the compound represented by formula (I) was weighed out and dissolved in 0.25 mL of water (solvent A), and the reaction mixture was stirred for 1 day. The solid was collected by centrifugation and dried to give the crystal form B of the compound of formula I.

**Table 6. Preparation of the crystal form B of the compound of formula I**

| NO | Formula I | Solvent A |
|---|---|---|
| 1 | | 0.25 mL of water |
| 2 | | 0.25 mL of methanol |
| 3 | | 0.25 mL of ethanol |
| 4 | | 0.25 mL of isopropanol |
| 5 | | 0.25 mL of n-propanol |
| 6 | | 0.25 mL of ethyl acetate |
| 7 | | 0.25 mL of acetonitrile |
| 8 | 5mg | 0.25 mL of isopropyl acetate |
| 9 | | 0.25 mL of methyl tert-butyl ether |
| 10 | | 0.25 mL of 2-butanone |
| 11 | | 0.25 mL of methyl isobutyl ketone |
| 12 | | 0.25 mL of n-heptane |
| 13 | | 0.25 mL of isopropyl ether |
| 14 | | 0.25 mL of isoamyl alcohol |
| 15 | | 0.25 mL of 10% water/methanol |
| 16 | | 0.25 mL of 7% water/ethanol |
| 17 | | 0.25 mL of 50% methanol/water |
| 18 | | 0.25 mL of 50% ethyl acetate/ethanol |
| 19 | | 0.25 mL of 50% ethyl acetate/n-heptane |
| 20 | | 0.25 mL of cyclohexane |
| 21 | | 0.25 mL of n-hexane |
| 22 | | 0.25 mL of toluene |

### Example 7: Preparation of Crystal Form C of Compound of Formula I

About 10 mg of the compound represented by formula (I) was weighed out. 0.2 mL of 50% methanol/acetonitrile or 0.2 mL of tetrahydrofuran was added, and a solution of ethanolamine in ethanol (2 mol/L, 10 µL) was added. 0.4 mL of methyl tert-butyl ether was added to the reaction mixture, and after 2 days of stirring, 0.8 mL of methyl tert-butyl ether was added. The mixture was stirred for 1 day with the temperature cycled between 50 °C and 5 °C (at a rate of 45 °C/h) and stirred at 25 °C for 2 days. After centrifugation and drying, the crystal form C of the compound of formula I was obtained.

X-ray powder diffraction analysis was performed. The XRPD pattern is shown in FIG. 4, and its characteristic peak positions are shown in Table 7. The TGA thermogram showed a weight loss of 4.34% before 110 °C and a weight loss of 8.22% from 110 °C to 195 °C. The DSC thermogram showed endothermic peaks at 106.12 °C and 177.29 °C.

**Table 7**

| Peak No. | 2θ value [° or degrees] | d[Å] | Relative intensity% |
|---|---|---|---|
| 1 | 5.702 | 15.48568 | 32.9 |
| 2 | 7.841 | 11.26688 | 12.0 |
| 3 | 10.074 | 8.77327 | 22.9 |
| 4 | 10.643 | 8.30573 | 64.8 |
| 5 | 11.463 | 7.71309 | 21.3 |
| 6 | 12.119 | 7.29713 | 10.4 |
| 7 | 15.320 | 5.77910 | 8.6 |
| 8 | 15.656 | 5.65550 | 10.5 |
| 9 | 16.738 | 5.29253 | 19.9 |
| 10 | 17.229 | 5.14274 | 100.0 |
| 11 | 18.213 | 4.86693 | 44.1 |
| 12 | 18.666 | 4.74977 | 31.0 |
| 13 | 19.430 | 4.56489 | 10.4 |
| 14 | 19.868 | 4.46524 | 9.4 |
| 15 | 20.900 | 4.24697 | 37.7 |
| 16 | 22.057 | 4.02664 | 16.7 |
| 17 | 22.422 | 3.96191 | 28.8 |
| 18 | 23.397 | 3.79909 | 20.4 |
| 19 | 23.968 | 3.70982 | 22.7 |
| 20 | 25.932 | 3.43317 | 3.2 |
| 21 | 26.605 | 3.34773 | 8.0 |
| 22 | 27.111 | 3.28647 | 8.0 |
| 23 | 27.852 | 3.20067 | 6.8 |
| 24 | 29.536 | 3.02186 | 7.4 |

### Example 8: Preparation of Crystal Form I of Sodium Salt of Compound of Formula I

About 10 mg of the compound represented by formula (I) was weighed out and dissolved in 0.2 mL of 50% ethanol/ethyl acetate (solvent A). A sodium hydroxide solution (2 mol/L, 10.5 µL) was added, and 0.8 mL of methyl tert-butyl ether was added. The reaction mixture was stirred for 2 days. After centrifugation and drying, the crystal form I of the sodium salt of the compound of formula I was obtained.

**Table 8. Preparation of the crystal form I of the sodium salt of the compound of formula I**

| NO | Formula I | Solvent A |
|---|---|---|
| 1 | 10 mg | 0.2 mL of 50% ethanol/ethyl acetate |
| 2 | | 0.2 mL of 50% methanol/acetonitrile |
| 3 | | 0.2 mL of tetrahydrofuran |

X-ray powder diffraction analysis was performed. The XRPD pattern is shown in FIG. 5, and its characteristic peak positions are shown in Table 9. According to ion chromatography analysis, the sodium ion content was 4.85%. The TGA thermogram showed a weight loss of 7.36% from 30 °C to 110 °C. The DSC thermogram showed endothermic peaks at 90.11 °C, 122.09 °C, and 210.70 °C and an exothermic peak at 145.47 °C.

DVS test results showed that the sample had a hygroscopic weight gain of about 6.31 % under normal storage conditions (i.e., 25 °C and 60% RH), a hygroscopic weight gain of about 7.08% under accelerated experimental conditions (i.e., 70% RH), and a hygroscopic weight gain of about 42.38% under extreme conditions (90% RH).

**Table 9**

| Peak No. | 2θ value [° or degrees] | d[Å] | Relative intensity% |
|---|---|---|---|
| 1 | 4.741 | 18.62306 | 12.6 |
| 2 | 6.446 | 13.70155 | 56.5 |
| 3 | 8.350 | 10.58013 | 100.0 |
| 4 | 8.847 | 9.98744 | 47.5 |
| 5 | 9.335 | 9.46650 | 44.4 |
| 6 | 9.807 | 9.01130 | 20.3 |
| 7 | 12.845 | 6.88628 | 45.7 |
| 8 | 13.328 | 6.63775 | 27.9 |
| 9 | 13.739 | 6.44032 | 28.7 |
| 10 | 15.728 | 5.63006 | 30.2 |
| 11 | 16.901 | 5.24181 | 46.0 |
| 12 | 17.874 | 4.95847 | 66.8 |
| 13 | 19.059 | 4.65280 | 12.2 |
| 14 | 19.805 | 4.47913 | 14.0 |
| 15 | 20.776 | 4.27208 | 12.8 |
| 16 | 21.490 | 4.13156 | 24.6 |
| 17 | 22.799 | 3.89739 | 8.9 |
| 18 | 23.607 | 3.76570 | 18.5 |
| 19 | 24.363 | 3.65055 | 12.2 |
| 20 | 25.022 | 3.55587 | 5.8 |
| 21 | 25.460 | 3.49569 | 17.0 |
| 22 | 25.773 | 3.45395 | 28.8 |
| 23 | 27.902 | 3.19505 | 13.8 |
| 24 | 31.558 | 2.83276 | 6.0 |
| 25 | 34.194 | 2.62014 | 9.0 |

### Example 9: Preparation of Crystal Form I of Sodium Salt of Compound of Formula I

About 10 mg of the compound represented by formula (I) was weighed out. 0.2 mL of 2-butanone or 0.2 mL of acetonitrile was added, and a solution of sodium hydroxide in ethanol (1 mol/L, 21 µL) was added. The reaction mixture was stirred for 1 day. After centrifugation and drying, the crystal form I of the sodium salt of the compound of formula I was obtained.

### Example 10: Preparation of Crystal Form II of Sodium Salt of Compound of Formula I

About 10 mg of the compound represented by formula (I) was weighed out. 0.2 mL of isopropanol or 0.2 mL of 1,4-dioxane was added, and a solution of sodium hydroxide in ethanol (1 mol/L, 21 µL) was added. The reaction mixture was stirred for 1 day. After centrifugation and drying, the crystal form II of the sodium salt of the compound of formula I was obtained.

X-ray powder diffraction analysis was performed. The XRPD pattern is shown in FIG. 6, and its characteristic peak positions are shown in Table 10. According to ion chromatography analysis, the sodium ion content was 4.75%. The TGA thermogram showed a weight loss of 2.18% from 30 °C to 120 °C. The DSC thermogram showed endothermic peaks at 179.08 °C and 223.40 °C.

DVS test results showed that the sample had a hygroscopic weight gain of about 6.35% under normal storage conditions (i.e., 25 °C and 60% RH), a hygroscopic weight gain of about 22.41% under accelerated experimental conditions (i.e., 70% RH), and a hygroscopic weight gain of about 56.98% under extreme conditions (90% RH).

**Table 10**

| Peak No. | 2θ value [° or degrees] | d[Å] | Relative intensity% |
|---|---|---|---|
| 1 | 6.771 | 13.04394 | 100.0 |
| 2 | 8.505 | 10.38756 | 3.4 |
| 3 | 11.446 | 7.72443 | 5.1 |
| 4 | 13.464 | 6.57097 | 50.4 |
| 5 | 13.970 | 6.33437 | 24.4 |
| 6 | 14.419 | 6.13780 | 20.0 |
| 7 | 15.593 | 5.67820 | 52.2 |
| 8 | 17.093 | 5.18342 | 1.0 |
| 9 | 18.044 | 4.91229 | 12.5 |
| 10 | 19.952 | 4.44663 | 10.2 |
| 11 | 20.313 | 4.36838 | 6.8 |
| 12 | 22.868 | 3.88578 | 3.5 |
| 13 | 24.260 | 3.66581 | 3.3 |
| 14 | 32.304 | 2.76901 | 3.9 |

### Example 11: Preparation of Crystal Form II of Sodium Salt of Compound of Formula I

About 5 mg of the crystal form I of the sodium salt of the compound represented by formula (I) was weighed out, and 0.2 mL of isopropanol or 0.2 mL of isopropyl acetate was added. The reaction mixture was stirred for 2 days. After centrifugation and drying, the crystal form II of the sodium salt of the compound of formula I was obtained.

### Example 12: Preparation of Crystal Form II of Sodium Salt of Compound of Formula I

About 5 mg of the crystal form I of the sodium salt of the compound represented by formula (I) was weighed out and dissolved in 0.2 mL of ethanol or 0.2 mL of n-propanol, and 1 mL of n-heptane was added. The reaction mixture was stirred for 5 days. After centrifugation and drying, the crystal form II of the sodium salt of the compound of formula I was obtained.

### Example 13: Preparation of Crystal Form III of Sodium Salt of Compound of Formula I

About 5 mg of the crystal form I of the sodium salt of the compound represented by formula (I) was weighed out, and 0.2 mL of 1,4-dioxane was added. The reaction mixture was stirred for 2 days. After centrifugation and drying, the crystal form III of the sodium salt of the compound of formula I was obtained.

X-ray powder diffraction analysis was performed. The XRPD pattern is shown in FIG. 7, and its characteristic peak positions are shown in Table 11.

**Table 11**

| Peak No. | 2θ value [° or degrees] | d[Å] | Relative intensity% |
|---|---|---|---|
| 1 | 5.639 | 15.65948 | 3.4 |
| 2 | 6.605 | 13.37234 | 100.0 |
| 3 | 10.311 | 8.57211 | 4.3 |
| 4 | 13.323 | 6.64009 | 3.4 |
| 5 | 17.914 | 4.94765 | 0.6 |
| 6 | 18.597 | 4.76739 | 13.0 |
| 7 | 19.295 | 4.59647 | 1.0 |
| 8 | 20.094 | 4.41548 | 8.9 |
| 9 | 20.676 | 4.29242 | 0.8 |
| 10 | 23.843 | 3.72899 | 0.8 |
| 11 | 25.069 | 3.54927 | 2.1 |
| 12 | 26.882 | 3.31392 | 8.4 |
| 13 | 29.710 | 3.00463 | 1.7 |

### Example 14: Preparation of Crystal Form IV of Sodium Salt of Compound of Formula I

About 2 mg of the crystal form I of the sodium salt of the compound represented by formula (I) was weighed out and heated to 180 °C to give the crystal form IV of the sodium salt of the compound of formula I. X-ray powder diffraction analysis was performed. The XRPD pattern is shown in FIG. 8, and its characteristic peak positions are shown in Table 12.

**Table 12**

| Peak No. | 2θ value [° or degrees] | d[Å] | Relative intensity% |
|---|---|---|---|
| 1 | 5.078 | 17.38829 | 28.9 |
| 2 | 7.078 | 12.47937 | 100.0 |
| 3 | 8.110 | 10.89305 | 12.6 |
| 4 | 10.670 | 8.28432 | 18.0 |
| 5 | 13.231 | 6.68637 | 23.3 |
| 6 | 14.227 | 6.22042 | 43.8 |
| 7 | 15.004 | 5.89984 | 31.1 |
| 8 | 17.071 | 5.18980 | 16.1 |
| 9 | 18.149 | 4.88389 | 5.7 |
| 10 | 18.812 | 4.71328 | 37.3 |
| 11 | 19.430 | 4.56489 | 10.1 |
| 12 | 20.081 | 4.41818 | 53.8 |
| 13 | 20.642 | 4.29935 | 17.6 |
| 14 | 21.451 | 4.13908 | 6.2 |
| 15 | 22.529 | 3.94340 | 12.1 |
| 16 | 24.018 | 3.70226 | 31.0 |
| 17 | 24.753 | 3.59398 | 7.5 |
| 18 | 27.144 | 3.28246 | 6.4 |
| 19 | 29.469 | 3.02861 | 3.6 |

### Example 15: Preparation of Amorphous Form of Sodium Salt of Compound of Formula I

About 2 mg of the crystal form I of the sodium salt of the compound represented by formula (I) was weighed out and heated to 130 °C to give the title product. The product was identified as an amorphous form of the sodium salt by X-ray powder diffraction. Its XRPD pattern is shown in FIG. 9.

### Example 16: Preparation of Crystal Form I of Ethanolamine Salt of Compound of Formula I

About 30 mg of the compound represented by formula (I) was weighed out, and 0.6 mL of tetrahydrofuran was added. A solution of ethanolamine in ethanol (2 mol/L, 31.5 µL) was added, and 3.6 mL of methyl tert-butyl ether was added. The reaction mixture was stirred for 3 days with the temperature cycled between 50 °C and 5 °C (at a rate of 45 °C/h). After centrifugation and drying, the crystal form I of the ethanolamine salt of the compound of formula I was obtained.

X-ray powder diffraction analysis was performed. The XRPD pattern is shown in FIG. 10, and its characteristic peak positions are shown in Table 13. The TGA thermogram showed a weight loss of 13.57% from 30 °C to 190 °C. The DSC thermogram showed endothermic peaks at 59.63 °C and 148.77 °C.

**Table 13**

| Peak No. | 2θ value [° or degrees] | d[Å] | Relative intensity% |
|---|---|---|---|
| 1 | 5.343 | 16.52676 | 38.3 |
| 2 | 9.612 | 9.19376 | 100.0 |
| 3 | 10.751 | 8.22273 | 32.1 |
| 4 | 12.023 | 7.35503 | 14.5 |
| 5 | 14.613 | 6.05694 | 10.7 |
| 6 | 16.737 | 5.29284 | 27.6 |
| 7 | 17.886 | 4.95534 | 16.3 |
| 8 | 19.178 | 4.62424 | 33.7 |
| 9 | 20.434 | 4.34263 | 12.2 |
| 10 | 21.788 | 4.07572 | 12.9 |
| 11 | 25.089 | 3.54647 | 5.0 |

### Example 17. Influencing Factor Stability Study

Samples of the free-state crystal forms A and B of the compound represented by formula I were spread in open containers and left to stand under light exposure (4500 Lux), high temperature (40 °C and 60 °C), and high humidity (RH 75% and RH 93%) conditions for 1 month to study their stability.

**Table 14. Influencing factors**

| **Conditions** | **Time (days)** | **Crystal form A** | | **Crystal form B** | |
|---|---|---|---|---|---|
| | | Purity% | Crystal form | Purity% | Crystal form |
| Initial | 0 | 98.6 | Crystal form A | 97.7 | Crystal form B |
| 40 °C | 7 days | 98.6 | Crystal form A | 97.7 | Crystal form B |
| | 14 days | 98.6 | Crystal form A | 97.7 | Crystal form B |
| | 1 month | 98.6 | Crystal form A | 97.7 | Crystal form B |
| 60 °C | 7 days | 98.6 | Crystal form A | 97.7 | Crystal form B |
| | 14 days | 98.6 | Crystal form A | 97.7 | Crystal form B |
| | 1 month | 98.6 | Crystal form A | 97.7 | Crystal form B |
| 75% RH | 7 days | 98.6 | Crystal form A | 97.7 | Crystal form B |
| | 14 days | 98.6 | Crystal form A | 97.7 | Crystal form B |
| | 1 month | 98.6 | Crystal form A | 97.7 | Crystal form B |
| 93% RH | 7 days | 98.6 | Crystal form A | 97.7 | Crystal form B |
| | 14 days | 98.6 | Crystal form A | 97.7 | Crystal form B |
| | 1 month | 98.6 | Crystal form A | 97.7 | Crystal form B |
| 4500 Lux | 7 days | 98.3 | Crystal form A | 97.7 | Crystal form B |
| | 14 days | 98.1 | Crystal form A | 97.5 | Crystal form B |
| | 1 month | 97.0 | Crystal form A | 97.0 | Crystal form B |

Conclusion: Standing for 1 month, the free-state crystal form A and the free-state crystal form B of the compound represented by formula I exhibited good physical and chemical stability.

Samples of the crystal forms I and II of the sodium salt of the compound represented by formula I were spread in open containers and left to stand under light exposure (4500 Lux), high temperature (40 °C and 60 °C), and high humidity (RH 75%) conditions for 1 month to study their stability.

**Table 15. Influencing factors**

| **Conditions** | **Time (days)** | **Crystal form I of sodium salt** | | **Crystal form II of sodium salt** | |
|---|---|---|---|---|---|
| | | Purity% | Crystal form | Purity% | Crystal form |
| Initial | 0 | 99.4 | Crystal form I | 98.9 | Crystal form II |
| 40 °C | 7 days | 99.4 | Crystal form I | 98.9 | Crystal form II |
| | 14 days | 99.4 | Crystal form I | 98.9 | Crystal form II |
| | 1 month | 99.3 | Crystal form I | 98.7 | Crystal form II |
| 60 °C | 7 days | 99.4 | Crystal form I | 98.8 | Crystal form II |
| | 14 days | 99.3 | Crystal form I | 98.7 | Crystal form II |
| | 1 month | 99.0 | Crystal form I | 98.6 | Crystal form II |
| 75% RH | 7 days | 99.4 | Crystal form I | 98.9 | Deliquesced |
| | 14 days | 99.4 | Crystal form I | 98.9 | Deliquesced |
| | 1 month | 99.4 | Crystal form I | 98.8 | Deliquesced |
| 4500 Lux | 7 days | 99.3 | Crystal form I | 98.9 | Crystal form II |
| | 14 days | 99.1 | Crystal form I | 98.8 | Crystal form II |
| | 1 month | 98.8 | Crystal form I | 98.6 | Crystal form II |

Conclusion: Standing for 1 month, the crystal form I of the sodium salt exhibited good physical and chemical stability, and the crystal form II of the sodium salt exhibited good physical and chemical stability under conditions other than high humidity.

### Example 18: Long-Term/Accelerated Stability

The free-state crystal form A and the free-state crystal form B of the compound represented by formula I were left to stand under conditions of 25 °C/60% RH and 40 °C/75% RH to study their stability.

**Table 16. Long-term acceleration**

| **Conditions** | **Time (days)** | **Free-state crystal form A** | | **Free-state crystal form B** | |
|---|---|---|---|---|---|
| | | Purity% | Crystal form | Purity% | Crystal form |
| Initial | 0 | 98.6 | Crystal form A | 97.7 | Crystal form B |
| 25 °C/60% RH | 1 month | 98.6 | Crystal form A | 97.7 | Crystal form B |
| | 2 months | 98.6 | Crystal form A | 97.7 | Crystal form B |
| | 3 months | 98.6 | Crystal form A | 97.7 | Crystal form B |
| | 6 months | 98.6 | Crystal form A | 97.7 | Crystal form B |
| 40 °C/75% RH | 1 month | 98.6 | Crystal form A | 97.7 | Crystal form B |
| | 2 months | 98.6 | Crystal form A | 97.7 | Crystal form B |
| | 3 months | 98.6 | Crystal form A | 97.7 | Crystal form B |
| | 6 months | 98.6 | Crystal form A | 97.7 | Crystal form B |

Conclusion: Standing under long-term accelerated conditions for 6 months, the free-state crystal form A and the free-state crystal form B exhibited good physical and chemical stability.

The crystal forms I and II of the sodium salt of the compound represented by formula I were left to stand under conditions of 25 °C/60% RH and 40 °C/75% RH to study their stability.

**Table 17. Long-term acceleration**

| **Conditions** | **Time (days)** | **Crystal form I of sodium salt** | | **Crystal form II of sodium salt** | |
|---|---|---|---|---|---|
| | | Purity% | Crystal form | Purity% | Crystal form |
| Initial | 0 | 99.4 | Crystal form I | 98.9 | Crystal form II |
| 25 °C/60% RH | 1 month | 99.4 | Crystal form I | 98.9 | Crystal form II |
| | 2 months | 99.4 | Crystal form I | 98.9 | Crystal form II |
| | 3 months | 99.4 | Crystal form I | 98.9 | Crystal form II |
| 40 °C/75% RH | 1 month | 99.4 | Crystal form I | 98.9 | Crystal form II |
| | 2 months | 99.4 | Crystal form I | 98.8 | Crystal form II |
| | 3 months | 99.4 | Crystal form I | 98.7 | Crystal form II |

Conclusion: Standing under long-term accelerated conditions for 3 months, the crystal forms I and II of the sodium salt exhibited good physical and chemical stability.

## Claims

1. A crystal form A of the compound represented by formula I: 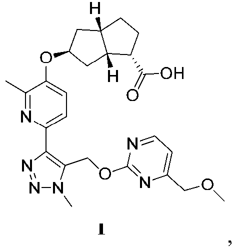 wherein an X-ray powder diffraction pattern expressed in terms of 2*θ* angles, which are diffraction angles, of the crystal form has characteristic peaks at 7.534, 10.223, 14.364, 17.269, 18.495, and 19.553, preferably at 7.534, 10.223, 14.364, 15.816, 16.678, 17.269, 18.495, and 19.553, and more preferably at 7.534, 10.223, 14.364, 15.816, 16.678, 17.269, 18.495, 19.553, 21.043, 21.342, 23.647, and 25.965; most preferably, the X-ray powder diffraction pattern expressed in terms of 2*θ* angles, which are diffraction angles, of the crystal form is shown in FIG. 2.

2. A crystal form B of the compound represented by formula I: 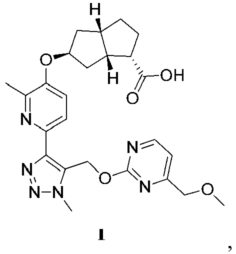 wherein an X-ray powder diffraction pattern expressed in terms of 2*θ* angles, which are diffraction angles, of the crystal form has characteristic peaks at 7.437, 10.381, 14.955, and 17.513, preferably at 7.437, 10.381, 14.717, 14.955, 17.513, 21.395, 22.607, and 23.221, and more preferably at 7.437, 10.381, 14.717, 14.955, 17.513, 18.466, 21.395, 21.692, 22.607, 23.221, and 25.972; most preferably, the X-ray powder diffraction pattern expressed in terms of 2*θ* angles, which are diffraction angles, of the crystal form is shown in FIG. 3.

3. A pharmaceutically acceptable salt of the compound represented by formula I, wherein the pharmaceutically acceptable salt is selected from the group consisting of a sodium salt and an ethanolamine salt:

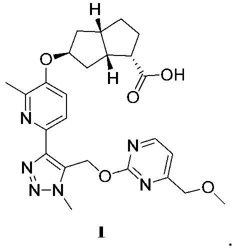

4. The pharmaceutically acceptable salt according to claim 3, wherein the chemical ratio of the compound represented by formula I to alkali ions is 1:1 or 1:2, preferably 1:1.

5. A preparation method for a pharmaceutically acceptable salt of the compound represented by I, comprising a step of reacting the compound represented by I with a base, wherein the base is selected from the group consisting of sodium hydroxide and ethanolamine:

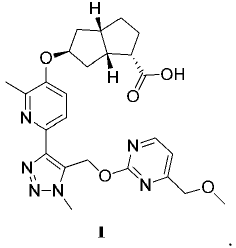

6. A crystal form I of a sodium salt of the compound represented by formula I: 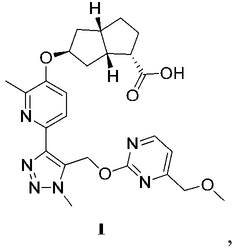 wherein an X-ray powder diffraction pattern expressed in terms of 2*θ* angles, which are diffraction angles, of the crystal form has characteristic peaks at 6.446, 8.350, 8.847, 9.335, 12.845, 16.901, and 17.874, preferably at 6.446, 8.350, 8.847, 9.335, 9.807, 12.845, 13.328, 13.739, 15.728, 16.901, and 17.874, more preferably at 4.741, 6.446, 8.350, 8.847, 9.335, 9.807, 12.845, 13.328, 13.739, 15.728, 16.901, 17.874, 19.059, and 19.805, and more preferably at 4.741, 6.446, 8.350, 8.847, 9.335, 9.807, 12.845, 13.328, 13.739, 15.728, 16.901, 17.874, 19.059, 19.805, 21.490, 23.607, 25.460, and 25.773; most preferably, the X-ray powder diffraction pattern expressed in terms of 2*θ* angles, which are diffraction angles, of the crystal form is shown in FIG. 5.

7. A crystal form II of a sodium salt of the compound represented by formula I: 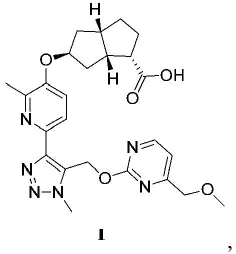 wherein an X-ray powder diffraction pattern expressed in terms of 2*θ* angles, which are diffraction angles, of the crystal form has characteristic peaks at 6.771, 13.464, 13.970, 14.419, 15.593, 18.044, and 19.952, preferably at 6.771, 11.446, 13.464, 13.970, 14.419, 15.593, 18.044, 19.952, and 24.260, and more preferably at 6.771, 8.505, 11.446, 13.464, 13.970, 14.419, 15.593, 18.044, 19.952, 20.313, 22.868, 24.260, and 32.304; most preferably, the X-ray powder diffraction pattern expressed in terms of 2*θ* angles, which are diffraction angles, of the crystal form is shown in FIG. 6.

8. A crystal form of the compound represented by formula I or a crystal form of a pharmaceutically acceptable salt of the compound represented by formula I, wherein 2*θ* angles have a margin of error of ±0.2:

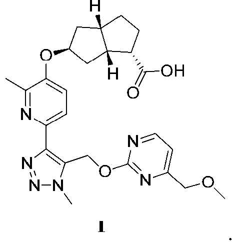

9. A pharmaceutical composition, comprising the following components:
i) the crystal form of the compound represented by formula I according to any one of claim 1 or 2, or the pharmaceutically acceptable salt of the compound represented by formula I according to any one of claim 3 or 4, or the crystal form according to claim 6 or 7; and
ii) one or more pharmaceutically acceptable excipients.

10. A method for preparing a pharmaceutical composition, comprising a step of mixing the crystal form of the compound represented by formula I according to any one of claim 1 or 2, or the pharmaceutically acceptable salt of the compound represented by formula I according to any one of claim 3 or 4, or the crystal form according to claim 6 or 7 with a pharmaceutically acceptable excipient.

11. Use of the crystal form of the compound represented by formula I according to any one of claim 1 or 2, or the pharmaceutically acceptable salt of the compound represented by formula I according to any one of claim 3 or 4, or the crystal form according to claim 6 or 7, or the composition according to claim 9 in the manufacture of an LPA1 inhibitor.

12. Use of the crystal form of the compound represented by formula I according to any one of claim 1 or 2, or the pharmaceutically acceptable salt of the compound represented by formula I according to any one of claim 3 or 4, or the crystal form according to claim 6 or 7, or the composition according to claim 9 in the manufacture of a medicament for treating and/or preventing fibrotic diseases of organs, respiratory diseases, renal diseases, hepatic diseases, inflammatory diseases, neurological diseases, cardiovascular and cerebrovascular diseases, gastrointestinal diseases, pain, urological diseases, ophthalmic diseases, metabolic diseases, cancer, and transplant rejection in patients.
